# EUROPEAN PATENT APPLICATION

(11) **EP 4 130 246 A1**
(43) Date of publication of application: **08.02.2023**
(21) Application number: 21776694.8
(22) Date of filing: 25.03.2021
(51) Int. Cl.: C12N 5/077, A23J 3/00, A23L 13/00, A61L 27/36

(54) **MUSCLE TISSUE PRODUCED BY BIOPRINTING**

(30) Priority: 26.03.2020 JP 2020056036
(71) Applicant: OSAKA UNIVERSITY, Suita-shi Osaka 565-0871 (JP)
(72) Inventor: MATSUSAKI, Michiya, Suita-shi, Osaka 565-0871 (JP); KANG, Donghee, Suita-shi, Osaka 565-0871 (JP)
(74) Representative: Hoffmann Eitle
(86) International application number: PCT/JP2021/014104
(87) International publication number: WO 2021/193980

(57) **Abstract**

[Problem] To provide a method for producing an artificial three-dimensional muscle tissue, said method enabling stable, efficient and easy production of a muscle tissue, and an artificial three-dimensional muscle tissue produced by this method. [Solution] A method for producing an artificial three-dimensional muscle tissue, said method comprising steps of (i) forming a three-dimensional muscle tissue precursor, which is configured from a first muscle tissue support, a second muscle tissue support and muscle cells, by linearly arranging the muscle cells on the first muscle tissue support in such a manner that the muscle cells are located close to the first muscle tissue support at one end of the line and close to the second muscle tissue support at the other end of the line, and (ii) culturing the three-dimensional muscle tissue precursor to give an artificial three-dimensional muscle tissue, and an artificial three-dimensional muscle tissue obtained by this method.

## Description

### Technical Field

The present invention relates to a method for producing an artificial three-dimensional muscular tissue and an artificial three-dimensional muscular tissue produced using the same.

### Background Art

As methods for producing artificial three-dimensional muscular tissues such as skeletal muscle, various methods have hitherto been known, including a multilayer three-dimensional cell culture scaffold system (Patent Literature 1). It is intended that artificial three-dimensional muscular tissues is used, for example, as muscle implants or the like for medical uses (Patent Literature 2). It is also intended that artificial three-dimensional muscular tissues is used, for example, for food such as processed meat (Patent Literature 3).

### Citation List

### Patent Literature

Patent Literature 1: Japanese Patent Laid-Open No. 2019-41755
Patent Literature 2: Japanese Patent Laid-Open No. 2003-508130
Patent Literature 3: Japanese Translation of PCT International Application Publication No. 2014-521336

### Summary of Invention

### Technical Problem

It is however difficult for conventional methods for producing artificial three-dimensional muscular tissues to produce muscular tissues stably and efficiently. A method for producing an artificial three-dimensional muscular tissue that easily produces muscular tissue stably and efficiently and an artificial three-dimensional muscular tissue produced using the same have therefore been required.

### Means of solving the problems

As a result of an earnest investigation to solve the above-mentioned problem, the present inventors have found a method for producing an artificial three-dimensional muscular tissue, comprising steps of: (i) disposing a first muscular tissue support, a second muscular tissue support, and muscle cells to constitute a three-dimensional muscular tissue precursor, wherein the muscle cells are disposed in the form of a line with one end of the line close to the first muscular tissue support and the other end of the line close to the second muscular tissue support, and (ii) culturing a three-dimensional muscular tissue precursor to obtain an artificial three-dimensional muscular tissue, and the present inventors have thus completed the present invention.

Specifically, the present invention provides the following.
[1] A method for producing an artificial three-dimensional muscular tissue, comprising steps of:
   (i) disposing a first muscular tissue support, a second muscular tissue support, and muscle cells to constitute a three-dimensional muscular tissue precursor, wherein the muscle cells are disposed in the form of a line with one end of the line close to the first muscular tissue support and another end of the line close to the second muscular tissue support, and
   (ii) culturing the three-dimensional muscular tissue precursor to obtain an artificial three-dimensional muscular tissue.
[2] The method for producing an artificial three-dimensional muscular tissue according to [1], wherein the step (i) comprises disposing the first muscular tissue support and the second muscular tissue support, and then disposing the muscle cells in the form of a line with the one end of the line close to the first muscular tissue support and the other end of the line close to the second muscular tissue support, to constitute the three-dimensional muscular tissue precursor.
[3] The method for producing an artificial three-dimensional muscular tissue according to [1], wherein the step (i) comprises disposing the muscle cells in the form of a line above the first muscular tissue support with the one end of the line close to the first muscular tissue support, and then disposing the second muscular tissue support with the other end of the line bought close to the second muscular tissue support, to constitute the three-dimensional muscular tissue precursor.
[4] The method according to any one of [1] to [3], wherein the muscle cells are disposed in a gel having thixotropy.
[5] The method according to any one of [1] to [4], wherein the muscle cells are disposed in the form of a line using a dispenser.
[6] The method according to [5], wherein the line of the muscle cells disposed imitates muscle fibers of an animal.
[7] The methods according to any one of [1] to [6], wherein the first muscular tissue support and/or the second muscular tissue support contains collagen.
[8] The method according to any one of [1] to [7], wherein the artificial three-dimensional muscular tissue is cultured with stretching tensile force applied to the artificial three-dimensional muscular tissue between the first muscular tissue support and the second muscular tissue support.
[9] The method according to any one of [1] to [8], wherein the artificial three-dimensional muscular tissue is for medical care, drug evaluation, or food.
[10] An artificial three-dimensional muscular tissue, comprising: a first muscular tissue support, a second muscular tissue support, and a muscular tissue, wherein the muscular tissue is linear, one end of the muscular tissue is connected to the first muscular tissue support, and another end of the muscular tissue is connected to the second muscular tissue support.
[11] The artificial three-dimensional muscular tissue according to [10], wherein the artificial three-dimensional muscular tissue has a sarcomere structure.
[12] The artificial three-dimensional muscular tissue according to [10] or [11], wherein the artificial three-dimensional muscular tissue is for medical care, drug evaluation, or food.

### Advantageous Effects of Invention

According to the present invention, an artificial three-dimensional muscular tissue can be produced stably and efficiently. The artificial three-dimensional muscular tissue can be prepared at high quality and/or low cost. The present invention eliminates the need to obtain muscular tissues from live animals.

The artificial three-dimensional muscular tissue produced by the present invention can be used for medical care. The artificial three-dimensional muscular tissue can be used, for example, as a muscle implant, a cardiac muscle sheet, or a part or the whole of an organ. According to the present invention, the artificial three-dimensional muscular tissue can be used for medical care without being obtained from a live animal.

The artificial three-dimensional muscular tissue produced by the present invention can be used for drug (medicine) evaluation. For example, an objective drug can be contacted with the artificial three-dimensional muscular tissue to evaluate the efficacy, the toxicity, or the like of the drug.

The artificial three-dimensional muscular tissue produced by the present invention can be used for food. For example, the artificial three-dimensional muscular tissue can be used as steak meat or processed meat. According to the present invention, edible meat can be obtained without killing animals such as domestic animals.

### Brief Description of Drawings

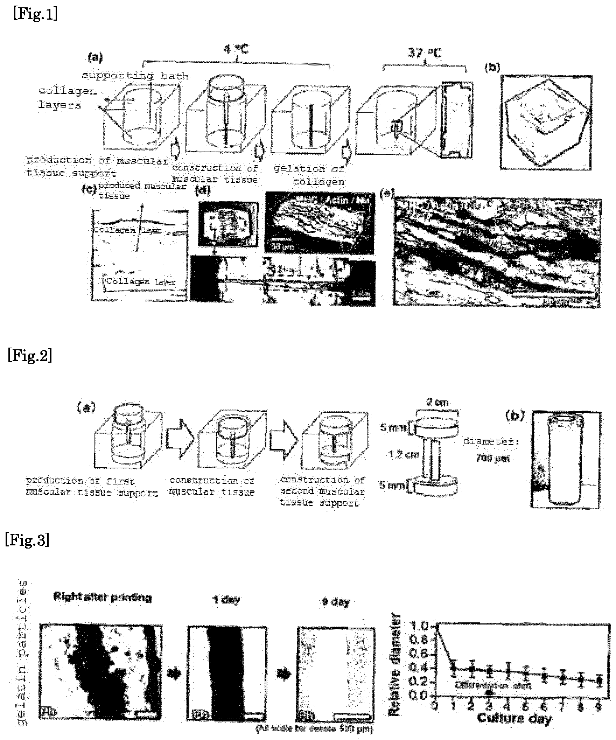

[Figure 1] Figure 1 (a) is a schematic diagram of muscular tissue production by bio-printing. Figure 1 (b) shows a printing container. Figure 1 (c) is a photograph after the printing. Figure 1 (d) shows a photograph after the culture of the produced muscular tissue for 1 day (upper left), a photograph of the connection between a collagen layer and the muscular tissue confirmed with a phase-contrast microscope (bottom), and a three-dimensional fluorescence image after differentiation (upper right, green: myosin heavy chains, red: actin filaments, and blue: cell nuclei). Figure 1 (e) shows a sarcomere structure seen in a fluorescence image of the immunostained muscular tissue two weeks after the differentiation (green: myosin heavy chains, red: actin filaments, and blue: cell nuclei).
[Figure 2] Figure 2 (a) is another schematic diagram of the muscular tissue production by bio-printing. Figure 2 (b) is a photograph after the printing.
[Figure 3] Figure 3 shows an image of muscular tissue produced using a supporting bath containing gelatin and the measurement results of the diameter of the muscular tissue.
[Figure 4] Figure 4 shows an image of muscular tissue produced using a supporting bath containing gellan gum and the measurement results of the diameter of the muscular tissue.
[Figure 5] Figure 5 shows a photograph of cultured meat prepared by assembling adipocytes, vascular cells, and produced muscular tissue.

### Description of Embodiments

In one embodiment, the present invention provides a method for producing an artificial three-dimensional muscular tissue, comprising steps of: (i) disposing a first muscular tissue support, a second muscular tissue support, and muscle cells to constitute a three-dimensional muscular tissue precursor, wherein the muscle cells are disposed in the form of a line with one end of the line close to the first muscular tissue support and the other end of the line close to the second muscular tissue support and (ii) culturing the three-dimensional muscular tissue precursor to obtain an artificial three-dimensional muscular tissue.

In one embodiment, the present invention provides a method for producing an artificial three-dimensional muscular tissue, comprising steps of: (i) disposing a first muscular tissue support and a second muscular tissue support; (ii) disposing muscle cells in the form of a line with one end of the line close to the first muscular tissue support and the other end of the line close to the second muscular tissue support, to constitute a three-dimensional muscular tissue precursor; and (iii) culturing the three-dimensional muscular tissue precursor to obtain an artificial three-dimensional muscular tissue.

In one embodiment, the present invention provides a method for producing an artificial three-dimensional muscular tissue, comprising steps of: (i) disposing muscle cells in the form of a line above a first muscular tissue support with one end of the line close to the first muscular tissue support; (ii) disposing a second muscular tissue support with the other end of the line close to the second muscular tissue support, to constitute a three-dimensional muscular tissue precursor; and (iii) culturing the three-dimensional muscular tissue precursor to obtain an artificial three-dimensional muscular tissue.

The muscle cells may be derived from any animal. The muscle cells may be muscle cells derived from a human, or may be muscle cells derived from an animal other than a human. The animal is, for example, an insect, a fish, an amphibian, a reptile, a bird, or a mammal. The animal is, for example, a frog, a chicken, a human, a monkey, a pig, a horse, a bovine, a sheep, a dog, a cat, a mouse, or a rabbit. The animal may be for meat, and a bovine, a pig, a sheep, a chicken, or the like is exemplified. The muscle cells may be cells derived from muscle, or may be muscle cells derived from stem cells such as mesenchymal stem cells, embryonic stem cells, and induced pluripotent stem cells. The muscle cells may contain other types of cells such as adipocytes, vascular endothelial cells, fibroblasts, stem cells, adipocytes, and vascular endothelial cells. The muscle cells may be cultured cells. Examples of the cultured cells include primary culture cells, subcultured cells, and cell line cells. The muscle cells may be myoblasts. The muscle cells may be any cells derived from a muscular tissue or a muscle tissue, for example, cells derived from skeletal muscle, visceral muscle, striated muscle, voluntary muscle, involuntary muscle, smooth muscle, or cardiac muscle.

The artificial three-dimensional muscular tissue contains an aggregate of cultured muscle cells, and, if desired, contains an extracellular matrix. The artificial three-dimensional muscular tissue may be like a skeletal muscular tissue, or may be like muscle tissue of an organ such as the heart. The artificial three-dimensional muscular tissue preferably forms muscle fiber. The artificial three-dimensional muscular tissue may have a sarcomere structure, which is the smallest constituent unit of muscle fiber. The artificial three-dimensional muscular tissue may have vasculature. The artificial three-dimensional muscular tissue may have three-dimensional structure disposed through an extracellular matrix. The artificial three-dimensional muscular tissue can be used, for example, as a muscle implant, a muscle sheet such as a cardiac muscle sheet, a part or the whole of an organ, or the like. The artificial three-dimensional muscular tissue can be used for drug (medicine) evaluation, namely the evaluation of the efficacy, the toxicity, or the like of an objective drug by contacting the drug with the artificial three-dimensional muscular tissue. The artificial three-dimensional muscular tissue can be used for food such as steak meat or processed meat.

The size and shape of the artificial three-dimensional muscular tissue can be suitably regulated depending on the use, the site of use, the type of muscle cells, physical properties necessary for muscular tissue, and the like. If the artificial three-dimensional muscular tissue is cylindrical, the diameter thereof may be, for example, several microns, several dozen microns, several hundred microns, several millimeters, several centimeters, or more. The height thereof may be, for example, several microns, several dozen microns, several hundred microns, several millimeters, several centimeters, or more.

The muscular tissue precursor is an aggregate of muscle cells before mature muscular tissue is formed. In one specific example, the muscular tissue precursor is a cell aggregate in which the first muscular tissue support and the second muscular tissue support are disposed close to both ends of the premature muscle cells disposed linearly.

The muscle cells may accompany an extracellular matrix. The extracellular matrix is well-known, and may be natural or artificial. The extracellular matrix may contain, for example, fibronectin, gelatin, collagen, laminin, poly-lysine, or the like. The extracellular matrix may be one, or may be two or more. The extracellular matrix preferably promotes or forms the intercellular adhesion between adjoining cells. A sheath structure can be formed in the artificial three-dimensional muscular tissue of the present invention by using the extracellular matrix.

A muscular tissue support is a base material to be used for constituting the three-dimensional muscular tissue precursor or for producing the artificial three-dimensional muscular tissue. The three-dimensional muscular tissue precursor, the first muscular tissue support, and the second muscular tissue support are integrated to produce the artificial three-dimensional muscular tissue. For example, the three-dimensional muscular tissue precursor that imitates muscle fibers, and the first muscular tissue support and the second muscular tissue support that imitate tendons are integrated to produce the artificial three-dimensional muscular tissue. Although, in the present invention, at least the first muscular tissue support and the second muscular tissue support are used as muscular tissue supports, a further support may be used.

Those skilled in the art can suitably determine the materials and sizes of the first muscular tissue support and the second muscular tissue support in view of the use of the artificial three-dimensional muscular tissue produced by integrating the three-dimensional muscular tissue precursor, the first muscular tissue support, the second muscular tissue support, and the like.

As long as the materials of the muscular tissue supports can be connected to the three-dimensional muscular tissue precursor, the materials of the muscular tissue supports may be any materials, and may be, for example, collagen (for example, one or more selected from the group consisting of type I, type II, type III, type IV, type V and type XI type). The materials of the muscular tissue supports can be suitably selected depending on the type or amount of the muscle cells, the size, shape, or physical properties of muscular tissue, or the like. The materials of the first muscular tissue support and the second muscular tissue support may be the same or different.

The sizes, shapes and physical properties of the muscular tissue supports can be also suitably selected depending on the type or amount of the muscle cells, the size, shape or physical properties of the muscular tissue, or the like. When the artificial three-dimensional muscular tissue is discoid, the diameter thereof is, for example, several microns, several dozen microns, several hundred microns, several millimeters, several centimeters, or more, and the thickness thereof is, for example, several microns, several dozen microns, several hundred microns, several millimeters, several centimeters, or more. The sizes and shapes of the first muscular tissue support and the second muscular tissue support may be the same or different.

The muscle cells can be disposed using well-known means and a well-known method. The muscle cells are typically disposed in gel.

The gel preferably has thixotropy. When the gel having thixotropy is used, the gel is liquefied by the nozzle motion and the cell discharge pressure from the nozzle, for example, at the time of disposing the muscle cells using the nozzle, and the degree of freedom of the size and shape of the three-dimensional muscular tissue precursor increases. After the disposition of the muscle cells, the gel having thixotropy solidifies, and the solidification is advantageous for maintaining the shape of the three-dimensional muscular tissue precursor and protecting the muscle cells. Alternatively, for example, the muscle cells may be disposed in the gel in a liquid state before the gelation, followed by subjecting the gel to gelation treatment. If the muscle cells are disposed in the gel in a liquid state, the nozzle moves easily. The gel subjected to gelation treatment after the disposition of the muscle cells is advantageous for maintaining the shape of the three-dimensional muscular tissue precursor and protecting the muscle cells.

The thixotropy refers to the fluid property characterized in that gel changes into sol by physical stimulus, for example, pressure, and that when the sol is left to stand, it returns to gel again (sol-gel transition). Specifically, the thixotropy has the phenomenon of the viscosity decreasing during the shear of liquid, the hysteresis found in the shear stress-shear rate curve of the liquid, and the phenomenon with a yield value.

The gel to be used for the present invention may be a gel obtained by dispersing a polymer substance (for example, a natural polymer such as a protein or a polysaccharide or a synthetic polymer) in a water-containing medium. Examples of the polymer constituting the gel include, but are not limited to, natural polymers such as agar, gelatin, agarose, xanthan gum, gellan gum, sclerotium gum, gum arabic, tragacanth gum, karaya gum, cellulose gum, tamarind gum, guar gum, locust bean gum, glucomannan, chitosan, carrageenan, quince seeds, galactan, mannan, starch, dextrin, curdlan, casein, pectin, collagen, fibrin, peptides, matrigel, chondroitin sulfate such as sodium chondroitin sulfate, hyaluronic acid (mucopolysaccharide) and hyaluronates such as sodium hyaluronate, alginic acid, alginates such as sodium alginate and calcium alginate, and derivatives thereof; cellulose derivatives such as methyl cellulose, hydroxymethyl cellulose, hydroxyethyl cellulose, hydroxypropyl cellulose, hydroxypropylmethyl cellulose, carboxymethyl cellulose, and salts thereof; poly(meth)acrylic acids such as polyacrylic acid, polymethacrylic acid, acrylic acid-alkyl methacrylate copolymers, and salts thereof; synthetic polymers such as polyvinyl alcohol, a polymer of polyethylene glycol di(meth)acrylate (PPEGDA and PPEGDM), polyhydroxyethyl methacrylate, polyacrylamide, poly(N,N-dimethylacrylamide), poly-2-acrylamide-2-methylpropanesulfonic acid, poly(N-isopropylacrylamide), polyvinylpyrrolidone, polystyrene sulfonate, polyethylene glycol, a carboxyvinyl polymer, an alkyl-modified carboxyvinyl polymer, a maleic anhydride copolymer, polyalkylene oxide-based resin, a crosslinked body of poly(methyl vinyl ether-alt-maleic anhydride) and polyethylene glycol, a crosslinked body of polyethylene glycol, a crosslinked body of N-vinylacetamide, a crosslinked body of acrylamide, and a crosslinked material of a starch-acrylate graft copolymer; silicone; interpenetrating network structural hydrogel and semi-interpenetrating network structural hydrogel (DN hydrogel); and mixtures of two or more thereof.

The gel viscosity can be regulated, or thixotropy can be imparted by regulating the amount (concentration) of the polymer in the medium. The amount (concentration) of the polymer in the medium can be regulated depending on the polymer type, the size, shape or physical properties of the three-dimensional muscular tissue precursor or the artificial three-dimensional muscular tissue, the size or motion speed of the nozzle to be used, and the like. The amount (concentration) of the polymer in the medium is preferably regulated so as to obtain the viscosity that enables maintaining the three-dimensional muscular tissue precursor or the artificial three-dimensional muscular tissue in the gel. The selection of the type and amount (concentration) of the polymer is within the skill of those skilled in the art.

Three-dimensional bio-printing may be used for disposing the muscle cells. The three-dimensional bio-printing is known. The three-dimensional bio-printing may be an automatic or semiautomatic process supported by a computer, or may be a manual process. A large amount of cells may be disposed at once using a dispenser having a nozzle, for example, a multi-nozzle dispenser. The nozzle caliber can be suitably selected depending on the size and shape of the desired artificial three-dimensional muscular tissue and the size and disposition of the cells.

The muscle cells may be disposed in imitation of muscle fiber of an animal. The muscle cells may be disposed in the form of one or more lines, for example, 2 or more, 3 or more, 4 or more, 5 or more, 10 or more, 20 or more, 30 or more, 40 or more, 50 or more, 100 or more, 500 or more, 1000 or more, 5000 or more, or 10000 or more lines. The muscle cells may be disposed so that a fascicle of muscle fibers is formed. The thickness (diameter) of the line that the muscle cells form may be 100 µm or more, 200 µm or more, 500 µm or more, 5mm or more, 10 mm or more, or 100 mm or more. The thickness (diameter) of the line that the muscle cells form may be 100 mm or less, 10 mm or less, 5 mm or less, 500 µm or less, 200 µm or less, or 100 µm or less. The length of the line that the muscle cells form may be 50 mm or more, 100 mm or more, or 200 mm or more. The muscle cells may be disposed in the form of a straight line. The muscle cells may be disposed in the form of a curved line. The line formed by the muscle cells may be one or more. Two or more (a plurality of) lines formed by the muscle cells can be cultured to reinforce the lines formed by the muscle cells.

The term "close" used herein refers to a state in which a small gap is present between muscle cells and a muscular tissue support or a state in which muscle cells and a muscular tissue support are in contact. The size of the small gap is a size such that when the muscle cells proliferate, they will be in contact with the muscular tissue support. Although the size of the small gap is different depending on the size or shape of the muscular tissue, the cell type, or culture conditions, the size may be, for example, several millimeters, several hundred microns, several dozen microns, several microns, or less. The muscle cells and the muscular tissue support are preferably in contact.

The term "in the form of a line" or "line" used herein means that the muscle cells are disposed linearly or in an almost linear state. For the almost linear state, real muscular tissue in an animal may be referred to. Alternatively, the almost linear state can also refer to a state in which stretching tensile force is generated between the first muscular tissue support and the second muscular tissue support, and the muscular tissue of the artificial three-dimensional muscular tissue. The "in the form of a line" or the "line" may be in the form of a straight line or a straight line. The "in the form of a line" or the "line" may be in the form of a curved line or a curved line.

When the muscle cells are disposed in the form of a line above the first muscular tissue support, this means that the angle between the first muscular tissue support and the disposed muscular tissue, for example, 1 degree to 90 degrees, 45 degrees to 90 degrees, 60 degrees to 90 degrees, 70 degrees to 90 degrees, 80 to 90 degrees, 85 to 90 degrees, 86 to 90 degrees, 87 to 90 degrees, 88 to 90 degrees, 89 to 90 degrees, or 90 degrees (right angle). The angle between the second muscular tissue support and the disposed muscular tissue is also preferably the above-mentioned angle. The angle between the first muscular tissue support and/or the second muscular tissue support, and muscular tissue is not limited to the above-mentioned angle depending on the shape of a desired artificial three-dimensional muscular tissue.

One specific example of the disposition of the muscle cells is as follows: a first muscular tissue support is placed at one end of a cylindrical gel, a second muscular tissue support is placed at the other end, and muscle cells are disposed in the form of a line in the gel using a multi-nozzle dispenser. At that time, the one end of the line of the muscle cells is brought close to the first muscular tissue support, and the other end of the line is brought close to the second muscular tissue support. Another specific example of the disposition of the muscle cells is as follows: a first muscular tissue support is placed in one end of a cylindrical gel, and muscle cells are disposed in the form of a line in the gel using a multi-nozzle dispenser. At that time, the one end of the line of the muscle cells is brought close to the first muscular tissue support. A second muscular tissue support is then disposed so as to be close to the other end of the line of the muscle cells. A still another specific example of the disposition of the muscle cells is as follows: muscle cells are disposed in the form of a line in a cylindrical gel using a multi-nozzle dispenser. At that time, the muscle cells are disposed so that both ends of the line of the muscle cells reach both circular bases of the gel cylinder, respectively, or are near the circular bases, respectively. The first muscular tissue support and the second muscular tissue support are then disposed on both circular bases of the gel cylinder. A yet another specific example of the disposition of muscle cells is as follows: a first muscular tissue support is printed in one end of a gel using a three-dimensional printer, a second muscular tissue support is printed at the other end, and muscle cells are disposed in the form of a line using a multi-nozzle dispenser. At that time, one end of the line of muscle cells is brought close to the first muscular tissue support, and the other end of the line is brought close to the second muscular tissue support. A series of these steps are performed in the same gel. A yet another specific example of the disposition of muscle cells is as follows: a first muscular tissue support is printed in a gel using a three-dimensional printer, and muscle cells are disposed in the form of a line thereabove using a multi-nozzle dispenser. At that time, one end of the line of the muscle cells is brought close to the first muscular tissue support. A second muscular tissue support is then printed using a three-dimensional printer so as to be brought close to the other end of the line of the muscle cells. A series of these steps are performed in the same gel. A yet another specific example of the disposition of muscle cells is as follows: muscle cells are disposed in the form of a line in a gel using a multi-nozzle dispenser. At that time, the muscle cells are disposed so that both ends of the line of the muscle cells reach both circular bases of the cylinder gel, respectively, or are near the circular bases, respectively. A first muscular tissue support and a second muscular tissue support are printed on both circular bases, respectively, of the gel using a three-dimensional printer. A series of these steps are performed in the same gel.

The constituted three-dimensional muscular tissue precursor is then cultured to obtain an artificial three-dimensional muscular tissue. Culture conditions can be suitably selected and changed depending on the type of the muscle cells, the number of the muscle cells, the size, shape, or physical properties of the muscular tissue (for example, elasticity or texture), or the like. The selection and change of such culture conditions are within the skill of those skilled in the art.

The three-dimensional muscular tissue precursor may be cultured in liquid medium. If the three-dimensional muscular tissue precursor is constituted in the gel containing medium components, the three-dimensional muscular tissue precursor may be taken out of the gel and cultured in liquid medium. Alternatively, the three-dimensional muscular tissue precursor constituted in the gel may be cultured in the gel. The gel may contain medium components and/or a component for promoting the differentiation induction of the muscle cells. The gel may be a gel that can be dissolved or decomposed by physical and/or chemical treatment to be removed.

In one specific example of the three-dimensional muscular tissue precursor, a three-dimensional muscular tissue precursor is constituted in a gel having thixotropy, and the three-dimensional muscular tissue precursor is then taken out of the gel and cultured in liquid medium. In a special specific example of the culture of a three-dimensional muscular tissue precursor, a three-dimensional muscular tissue precursor is constituted in a gel having thixotropy and containing medium components and/or a component for promoting the differentiation induction of the muscle cells, the gel is then dissolved by physical and/or chemical treatment, and the three-dimensional muscular tissue precursor is cultured in the dissolved gel. Although examples of the physical or chemical treatment includes, but are not limited to, the addition of a gel resolvent, pH change, temperature change, and concussion. For example, gellan gum gel can be dissolved by adding tris buffer solution.

It is preferable that stretching tensile force be generated between the first muscular tissue support and/or the second muscular tissue support and the muscular tissue in the artificial three-dimensional muscular tissue of the present invention. Although the stretching tensile force may be generated in the step of culturing the three-dimensional muscular tissue precursor, the stretching tensile force may be generated before and/or after the culture step. The generation of the stretching tensile force facilitates the formation of the muscle fiber, the muscular tissue, or the sarcomere structure, and the obtained muscular tissue can resemble animal muscle. The stretching tensile force may be generated by the action of the muscle cells, or may be externally applied. The magnitude of the stretching tensile force can be suitably changed depending on the type of the muscle cells in the artificial three-dimensional muscular tissue, the number of the muscle cells, the size, shape, or physical properties (for example, elasticity or texture) of the muscular tissue, or the like.

In the step of culturing the three-dimensional muscular tissue precursor, or before and/or after the culture step, differentiation of the muscle cells may be induced, and/or the muscular tissue may be matured. The method for inducing differentiation of the muscle cells and that for maturing the muscular tissue are known. For example, the component for promoting induction of the differentiation of the muscle cells may be added to the medium. The muscular tissue may be matured, for example, using electrical stimulation. The medium components for cell culture and the component for inducing differentiation of the muscle cells are known, and can be suitably selected.

The artificial three-dimensional muscular tissue obtained by the production method of the present invention can be for food. "For food" used herein means that the artificial three-dimensional muscular tissue is suitable to be eaten by a human or an animal other than a human. The artificial three-dimensional muscular tissue of the present invention can be used for food such as steak or processed meat. Proteins, fats, carbohydrates, vitamins, minerals, fiber, fatty acids, amino acids, flavor materials, coloring materials, and the like may be added to the artificial three-dimensional muscular tissue so that the artificial three-dimensional muscular tissue is suitable for food, for example, in view of regulating the texture, nutritive values, the taste, the color, and the like. The shape, weight, size, or the like of the artificial three-dimensional muscular tissue may be suitably regulated so that the artificial three-dimensional muscular tissue is suitable for food.

In a further embodiment, the present invention provides an artificial three-dimensional muscular tissue, comprising: a first muscular tissue support, a second muscular tissue support, and a muscular tissue, wherein the muscular tissue is linear, one end of the muscular tissue is connected to the first muscular tissue support, and the other end of the muscular tissue is connected to the second muscular tissue support.

The artificial three-dimensional muscular tissue of the present invention may have a sarcomere structure. The artificial three-dimensional muscular tissue of the present invention may have vasculature. The artificial three-dimensional muscular tissue of the present invention may have a three-dimensional structure disposed through an extracellular matrix.

The artificial three-dimensional muscular tissue of the present invention may be for medical care, drug evaluation, or food.

The terms used herein is terms usually used in the art unless otherwise specified.

Although the present invention will be described further specifically and in detail by way of Examples hereinafter, it should not be understood that the scope of the present invention is limited to these Examples.

### Examples

### Example 1

### 1. Preparation of collagen nanofiber solution

First, 1 wt% collagen sponge (produced by NH Foods Ltd.) was mixed in a PBS 1x solution to prepare a collagen microfiber solution using a crusher (VH-10 homogenizer manufactured by AS ONE CORPORATION). The prepared collagen microfiber solution was then kept in a refrigerator at 4°C for several hours to prepare a collagen nanofiber (CFN) solution.

### 2. Production of supporting bath

First, 4.5 wt% of gelatin was mixed in DMEM containing 10% of FBS and 1% of antibiotics (antibiotics/NACALAI TESQUE, INC. /02892-54), and the gelatin was dissolved at 37°C. After the dissolution, the resultant was gelated in a refrigerator at 4°C. The gelatin gel was mixed with DMEM (10% FBS and 1% antibiotics) solution having the same volume as the gelatin gel, and crushed with a crusher. DMEM (10% FBS and 1% antibiotics) having the same volume as the crushed gelatin dispersion was added to the gelatin dispersion, and the mixture was centrifuged (4200 rpm/3 minutes). After the removal of the supernatant, thrombin was added so that the concentration of the thrombin was 10 U/mL.

### 3. Provision of printing container

The printing container was made of a PDMS material as seen in Figure 1 (b). Regarding the internal size, the area of the base was 1 cm² (1 cm × 1 cm), and the height was 2 cm. Collagen layers was each 0.4 cm high, and a supporting bath region was 0.8 cm high.

### 4. Cell culture

### 4-1 Proliferation of muscle satellite cells derived from bovine.

Muscle satellite cells isolated from tissue of a bovine were cultured in a cell culture flask (3-layer cell culture flask/Falcon/353143). The method for proliferation subculture of the satellite cells was performed in the same protocol as that for common adherent cells. To high glucose DMEM (DMEM/Thermo Fisher Scientific K.K. /10569010) were added 20% of FBS (FBS/Corning/35-010-CV), 1% of antibiotics, 4 ng/mL of bFGF, and 10 um/mL of p38 inhibitor (p38 inhibitor/selleck/S1076) to prepare culture medium.

### 4-2. Differentiation of muscle satellite cells derived from bovine

After culture medium exchange, differentiation of the muscle satellite cells were induced. The culture medium was changed for DMEM obtained by adding 2% of horse serum and 1% of antibiotics to conventional culture medium for proliferation. The culture medium was changed every 2 to 3 days.

### 5. Cell printing

### 5-1. Production of printing bath

First, around 0.4 ml of 1 wt% collagen nanofiber solution described in Example 1.1 was poured into the bottom region of the printing container described in Example 1.3 and gelated at 37°C for around 2 hours. A supporting bath region was filled with 4.5 wt% gelatin particles including 10 U/mL thrombin mixed, above the gelated collagen gel in the bottom region. Then, 0.4 mL of the collagen nanofiber solution were further poured into the region above the supporting bath region.

### 5-2 Preparation of bioink

The muscle satellite cells were separated on a culture plate and then prepared to a concentration of 5 × 10⁷ cells/ml. Then, 20 mg/mL fibrinogen was mixed with the cells (60% of the whole amount), and the matrigel in an amount of the remaining 40% of the whole amount was additionally mixed to prepare a bioink. The mixing process was performed on ice at 4°C to minimize the gelation of matrigel.

### 5-3 Printing

The printing container in which the supporting bath material and the collagen layers were formed as described in Example 5.1 was placed on the substrate of a printing device maintained at 4°C (manufactured by Musashi Engineering, Inc., NANO MASTER) and left to stand for around 10 minutes so that the collagen layers in the bottom regions was liquid. The prepared bioink was inserted into a syringe, and the syringe was installed in the printing device. A 16G nozzle (1.19 mm in inner diameter and 1.65 mm in outer diameter) was used, and the cells were printed at traveling speed of 1 mm/s over 1.5 cm in the height direction of the printing container.

### 5-4 Subsequent operation

After the cell printing, additional collagen solution was poured into the region where the nozzle passed from the collagen layer in the upper region, and the temperature was then maintained at room temperature for 30 minutes. Subsequently, the additional collagen solution was also poured into the collagen layer in the floor region, and the temperature was then maintained at room temperature for 30 minutes. Thereafter, the container was kept in an incubator at 37°C for 2 hours to gelate the collagen. Meanwhile, the supporting bath was liquefied. After the gelation of the collagen, additional culture medium (High glucose DMEM, 20% FBS, 1% antibiotics, and 4ng/mL bFGF) was added to the printing container performed in the PDMS for culture.

### 6. Confirmation of muscular tissue after printing 6-1. Induction of differentiation

The existing culture medium (High glucose DMEM, 20% FBS, 1% antibiotics, and 4 ng/ml bFGF) was exchanged for culture medium (High glucose DMEM, 2% horse serum, and 1% antibiotics) for differentiation induction to induce differentiation into muscular fiber.

### 6-2. Immunostaining and fluorescence image photography

The cultured muscular tissue was fixed in 4% paraformaldehyde at room temperature for 20 minutes and then washed with PBS 1x. Subsequently, the tissue was treated with 0.2 wt% Triton X-100 for 15 minutes and then washed with PBS 1x. After treatment with 1 wt% BSA at room temperature for 30 minutes, the muscular tissue was subjected to primary staining with MF20 (myosin 4 monoclonal antibody) prepared at 1 µg/ml in 1 wt% BSA at room temperature for 2 hours. After washing with PBS 1x, fluorescence conjugated phalloidin and a second antibody corresponded to the primary staining were mixed in 1% BSA for treatment at room temperature for 1 hour. After the washing with PBS 1x, the nuclei were stained with DAPI (4',6-diamidino-2-phenylindole dihydrochloride), and three-dimensional fluorescence photography was then performed using a confocal laser microscope. The photographed fluorescence images can be confirmed in Figures 1 (d) and (e).

### 6-3. Measurement of muscle fiber diameter

The measurement results of the cultured muscular tissue diameter were also obtained (Figure 3).

### Example 2

### 1. Preparation of collagen nanofiber solution

First, 1 wt% collagen sponge (produced by NH Foods Ltd.) was mixed in PBS 1x solution to prepare a collagen microfiber using a fiberizer (homogenizer). Then, the prepared collagen microfiber was kept in a refrigerator at 4°C for several hours to prepare a collagen nanofiber (CFN) solution.

### 2. Production of supporting bath

First, 0.1 g of gellan gum was dissolved in 10 mL of PBS at 100°C and the resultant was then gelated by incubation in a refrigerator at 4°C. The gelated gellan gum was pulverized into particles using a crusher. After centrifugation at 4200 rpm for 3 minutes, the supernatant was removed. Thrombin was added to the particulate gellan gum gel so that the concentration of the thrombin was 10 U/mL.

### 3. Preparation of the bioink

Then, 20 mg of fibrinogen was dissolved in 1 mL of DMEM at 37°C to prepare 20 mg/mL fibrinogen solution. At the time of cell printing, cells and extracellular matrix material were added.

### 4. Printing

The collagen nanofiber solution described in Example 2.1 and the bioink described in Example 2.3 were inserted into syringes separately. The syringe in which collagen nanofiber was inserted was first loaded on a printing device, and 500 µL was discharged into a 3 mL vial. The collagen nanofiber was gelated at 37°C for 2 hours. After the gelation, 2.5 mL of the prepared supporting bath described in Example 2.2 was added above the collagen gel. The syringe was replaced with the syringe for bio-printing, and printing was then performed under the following conditions: nozzle: 19G (0.68 mm in inner diameter, 1.00 mm outer diameter) and traveling speed: 1 mm/s. After the printing, fibrinogen gelation was accelerated at 37°C for 15 minutes. Figure 2 (b) shows the results.

### 5. Measurement of muscle fiber diameter

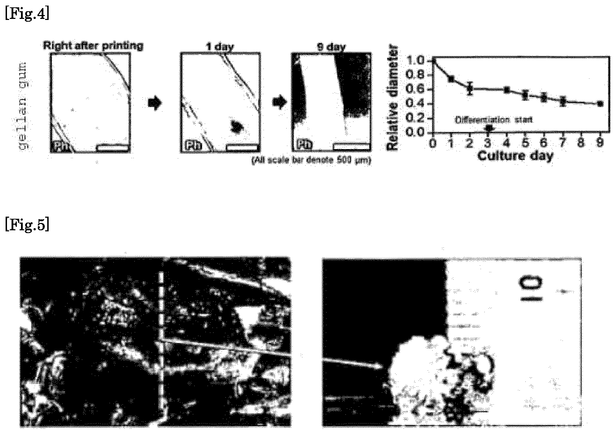

The measurement results of the cultured muscular tissue diameter were also obtained (Figure 4).

### Example 3

### 1. Preparation of cultured meat

Adipocytes, vascular cells, and muscular tissue obtained in Example 1 were assembled based on the ratio between the fat tissue, vascular tissue, and muscular tissue of commercial beef. Figure 5 shows the assembled cultured meat. The vascular cells and the muscular tissue are stained with carmine dye (red), and the adipocytes are not stained.

### Industrial Applicability

The present invention is useful in the field of therapeutic materials for regenerative medicine, wound healing, the recovery, maintenance, and improvement of motor function, and the like; the field of medicaments; the research field about muscle; the field of food and feed; and the like.

## Claims

1. A method for producing an artificial three-dimensional muscular tissue, comprising steps of:
(i) disposing a first muscular tissue support, a second muscular tissue support, and muscle cells to constitute a three-dimensional muscular tissue precursor, wherein the muscle cells are disposed in a form of a line with one end of the line close to the first muscular tissue support and another end of the line close to the second muscular tissue support, and
(ii) culturing the three-dimensional muscular tissue precursor to obtain an artificial three-dimensional muscular tissue.

2. The method for producing an artificial three-dimensional muscular tissue according to claim 1, wherein the step (i) comprises disposing the first muscular tissue support and the second muscular tissue support, and then disposing the muscle cells in the form of a line with the one end of the line close to the first muscular tissue support and the other end of the line close to the second muscular tissue support, to constitute the three-dimensional muscular tissue precursor.

3. The method for producing an artificial three-dimensional muscular tissue according to claim 1, wherein the step (i) comprises disposing the muscle cells in the form of a line above the first muscular tissue support with the one end of the line close to the first muscular tissue support, and then disposing the second muscular tissue support with the other end of the line close to the second muscular tissue support, to constitute the three-dimensional muscular tissue precursor.

4. The method according to any one of claims 1 to 3, wherein the muscle cells are disposed in a gel having thixotropy.

5. The method according to any one of claims 1 to 4, wherein the muscle cells are disposed in the form of a line using a dispenser.

6. The method according to claim 5, wherein the line of the muscle cells disposed imitates muscle fibers of an animal.

7. The methods according to any one of claims 1 to 6, wherein the first muscular tissue support and/or the second muscular tissue support contains collagen.

8. The method according to any one of claims 1 to 7, wherein the artificial three-dimensional muscular tissue is cultured with stretching tensile force applied to the artificial three-dimensional muscular tissue between the first muscular tissue support and the second muscular tissue support.

9. The method according to any one of claims 1 to 8, wherein the artificial three-dimensional muscular tissue is for medical care, drug evaluation, or food.

10. An artificial three-dimensional muscular tissue, comprising: a first muscular tissue support, a second muscular tissue support, and a muscular tissue, wherein the muscular tissue is linear, one end of the muscular tissue is connected to the first muscular tissue support, and another end of the muscular tissue is connected to the second muscular tissue support.

11. The artificial three-dimensional muscular tissue according to claim 10, wherein the artificial three-dimensional muscular tissue has a sarcomere structure.

12. The artificial three-dimensional muscular tissue according to claim 10 or 11, wherein the artificial three-dimensional muscular tissue is for medical care, drug evaluation, or food.
